# EUROPÄISCHE PATENTSCHRIFT

(11) **EP 2 683 695 B1**
(45) Veröffentlichungstag und Bekanntmachung des Hinweises auf die Patenterteilung: **05.08.2015**
(21) Anmeldenummer: 12707584.4
(22) Anmeldetag: 06.03.2012
(51) Int. Cl.: C07D 235/02

(54) **CIS-ALKOXYSUBSTITUIERTE SPIROCYCLISCHE 1-H-PYRROLIDIN-2,4-DION-DERIVATE**
CIS-ALKOXY-SUBSTITUTED SPIROCYCLIC 1-H-PYRROLIDINE-2,4-DIONE DERIVATIVES
DÉRIVÉS DE 1-H-PYRROLIDINE-2,4-DIONE SPIROCYCLIQUES À SUBSTITUTION CIS-ALKOXY

(30) Priorität: 11.03.2011 EP 11157904; 11.03.2011 US 201161451783 P
(43) Veröffentlichungstag der Anmeldung: 15.01.2014
(73) Patentinhaber: Bayer Intellectual Property GmbH, 40789 Monheim (DE)
(72) Erfinder: SCHNATTERER, Albert, 51373 Leverkusen (DE); HIMMLER, Thomas, 51519 Odenthal (DE); FISCHER, Reiner, 40789 Monheim (DE)
(74) Vertreter: BIP Patents
(86) Internationale Anmeldenummer: PCT/EP2012/053810
(87) Internationale Veröffentlichungsnummer: WO 2012/123287

(56) Entgegenhaltungen:
- WO-A1-02/02532

## Beschreibung

Die vorliegende Erfindung betrifft Verfahren zur Herstellung von cis-alkoxysubstituierten spirocyclischen 1-H-Pyrrolidin-2,4-dion-Derivaten und deren Alkali- und Erdalkalisalze.

Alkoxysubstituierte spirocyclische 1-H-Pyrrolidin-2,4-dion-Derivate der allgemeinen Formel (I) mit akarizider, insektizider und herbizider Wirkung sind bekannt: EP-A 596 298, WO95/20572, WO 95/26954, WO 95/20572, EP-A 668 267, WO 96/25395, WO 96/35664, WO 97/01535, WO 97/02243, WO 97/36868, WO 98/05638, WO 99/43649, WO 99/48869, WO 99/55673, WO 01/74770, WO 01/96333, WO 03/035643, WO 04/007448, WO 04/065366, WO 04/111042, WO 05/066125, WO 05/049569, WO 05/044796, WO 05/092897, WO 06/056282, WO 06/056281, WO 06/029799, WO 07/096058, WO 08/067910, WO 08/138551, WO 10/102758.

Solche Verbindungen werden üblicherweise ausgehend von den entsprechenden cis-substituierten Hydantoinen der Formel (cis-II) hergestellt.

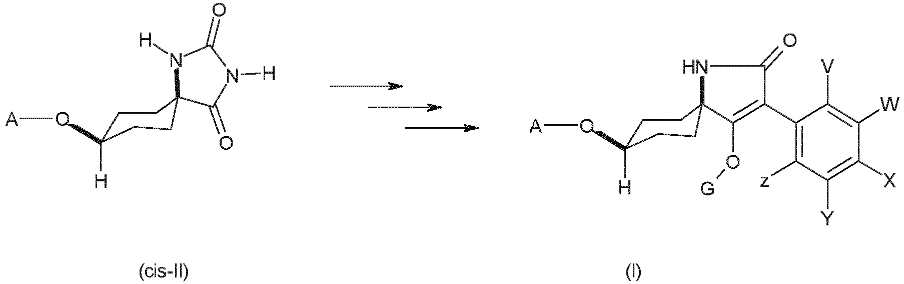

In den Formeln (I), (II) und (cis-II) stehen
- V,W,X,Y und Z: unabhängig voneinander für Wasserstoff, Alkyl, Halogen, Alkoxy, Halogenalkyl oder Halogenalkoxy,
- A: für C₁-C₆-Alkyl,
- G: für Wasserstoff (a) oder für eine der Gruppen 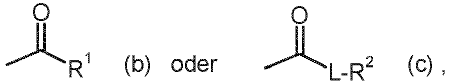 worin
- L: für Sauerstoff oder Schwefel,
- R¹: für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoiy substituiertes C₃-C₈-Cycloalkyl, in welchem gegebenenfalls eine oder zwei nicht direkt benachbarte Methylengruppen durch Sauerstoff und/oder Schwefel ersetzt sind, für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl oder für Thienyl,
- R²: für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl, für gegebenenfalls durch Halogen, C₁-C₆-Alkvl oder C₁-C₆-Alkoxy substituiertes C₃-C₈-Cycloalkyl oder für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.

Es ist bereits bekannt geworden, dass insbesonders die cis-substituierten Verbindungen der Formel (I) biologisch vorteilhafte Eigenschaften (Wirksamkeit, toxikologisches Profil) aufweisen (WO2004/007448). Daher besteht ein erhöhter Bedarf an den cis-substituierten Hydantoinen der Formel (cis-II) als Ausgangsmaterialien.

Bedingt durch die Herstellungsverfahren fallen die bekannten Verbindungen der Formel (II) in der Regel jedoch in Form von cis/trans Isomerengemischen an.

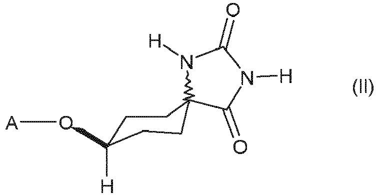

Es ist auch bereits bekannt geworden (WO2002/02532), aus einem Isomerengemisch der Formel (II) das gewünschte cis-Isomer (cis-II) in hoher Reinheit dadurch zu erhalten, dass man das Isomerengemisch mit wässrigem Ammoniak verrührt, wobei offenbar die Ammoniumsalze der Formel (III) entstehen. Da die cis-Salze (cis-III) eine deutlich geringere Löslichkeit als die trans-Salze (trans-III) ausweisen, können die Salze (cis-III) bzw. die Hydantoine (cis-II) durch Filtration in hoher Reinheit isoliert werden.

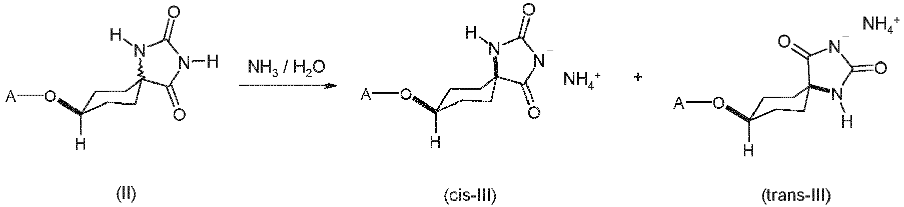

Dieses Verfahren hat jedoch mehrere Nachteile: Die Menge an Ammoniak, die verwendet werden muß, ist hoch (mehrfacher molarer Überschuß); die Verwendung von Ammoniak führt zu einer drastisch erhöhten Stickstoffbelastung des Abwassers, die unerwünscht ist und unter Umständen aufwendige Aufbereitungsmethoden für das Abwasser notwendig macht; die Basenstärke von Ammoniak ist für die im anschließenden Schritt folgende Hydrolyse des Hydantoins unter technisch akzeptablen Temperatur- und Druckbedingungen nicht ausreichend, so dass eine direkte Weiterverarbeitung von (cis-III) nicht möglich ist.

Es bestand also weiterhin Bedarf an einem einfacheren, technisch besser realisierbaren und umweltfreundlicheren Verfahren zur Isomerentrennung von Verbindungen der Formel (II).

Es wurde nun ein neues Verfahren zur Isolierung von cis-Hydantoinen der Formel (cis-II) gefunden, dadurch gekennzeichnet, dass man ein Isomerengemisch der Formel (II) mit einer wässrigen Lösung eines Alkali- oder Erdalkalihydroxids verrührt und anschließend das cis-Isomer isoliert.

Dabei erhält man, je nach Wahl des Alkali- oder Erdalkalihydroxids und der Bedingungen der Isolierung (Temperatur, Waschen des Feststoffes mit Wasser) Verbindungen (cis-II), die entsprechenden Salze (cis-IV) oder Gemische aus (cis-II) und (cis-IV).

Ebenfalls Gegenstand der vorliegenden Erfindung sind neue Alkali- und Erdalkalisalze der Formeln (cis-IV) und (trans-IV), die stark unterschiedliche Löslichkeiten in Wasser aufweisen und dadurch eine Isolierung von (cis-IV) bzw. (cis-II) ermöglichen. Die Verbindungen der Formeln (cis-IV) bzw. (trans-IV) können auch als entsprechende Hydrate vorliegen, die ebenfalls Gegenstand der Erfindung sind.

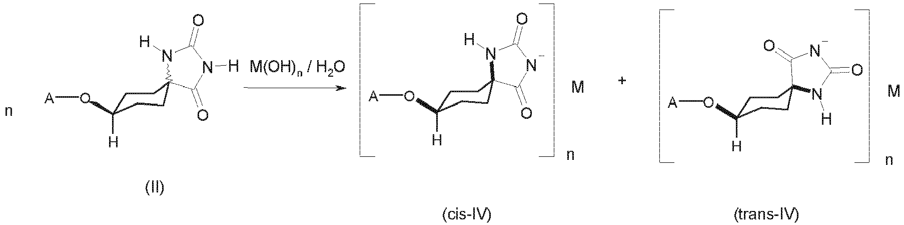

Hierbei stehen in den Formeln (IV)
- A: für C₁-C₆-Alkyl,
- M: für ein Alkalikation oder für ein Erdalkalikation,
und
- n: für 1, wenn M ein Alkalikation ist
oder
für 2, wenn M ein Erdalkalikation ist.

Die anderen möglichen mesomeren Strukturen für die Verbindungen der Formel (IV) sollen von dieser mit umfasst sein.

Bevorzugt steht
- A: für C₁- C₄-Alkyl,
- M: für ein Alkalikation
und
- n: für 1.

Besonders bevorzugt steht
- A: für Methyl, Ethyl, Propyl, i-Propyl, Butyl oder i-Butyl, (hervorgehoben für Methyl),
- M: für Li⁺, Na⁺ oder K⁺
und
- n: für 1.

Bei den in den vorstehenden Formeln angegebenen Definitionen der Symbole wurden Sammelbegriffe verwendet, die allgemein repräsentativ für die folgenden Substituenten stehen:
Alkyl: gesättigte, geradkettige oder verzweigte Kohlenwasserstoffreste mit 1 bis 6 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im Einzelnen seien außer den bei den Herstellungsbeispielen genannten Verbindungen die folgenden Verbindungen der Formel (cis-IV) genannt:

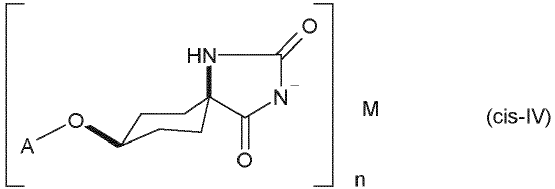

**Tabelle 1**

| **A** | **M** | **n** |
|---|---|---|
| C₂H₅ | Li⁺ | 1 |
| C₂H₅ | Na⁺ | 1 |
| C₂H₅ | K⁺ | 1 |
| C₂H₅ | Rb⁺ | 1 |
| C₂H₅ | Be²⁺ | 2 |
| C₂H₅ | Mg²⁺ | 2 |
| C₂H₅ | Ca²⁺ | 2 |
| C₂H₅ | Sr²⁺ | 2 |
| C₂H₅ | Ba²⁺ | 2 |
| C₃H₇ | Na⁺ | 1 |
| C₃H₇ | K⁺ | 1 |
| iC₃H₇ | Na⁺ | 1 |
| iC₃H₇ | K⁺ | 1 |
| C₄H₉ | Na⁺ | 1 |
| C₄H₉ | K⁺ | 1 |
| iC₄H₉ | Na⁺ | 1 |
| iC₄H₉ | K⁺ | 1 |

Die beim erfindungsgemäßen Verfahren als Ausgangsstoffe benötigten Verbindungen der Formel (II) sind bekannt und können nach bekannten Methoden (Strecker-Synthese; Bucherer-Bergs-Reaktion) hergestellt werden.

Man erhält die Verbindungen der Formel (cis-IV) bzw. (cis-II), indem man Hydantoine der Formel (II) 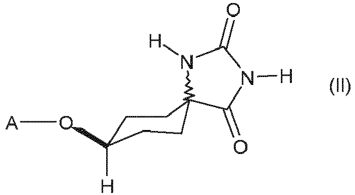 in welcher
- A: die oben angegebenen Bedeutungen hat,

in einem Verdünnungsmittel mit einem Alkali- oder Erdalkalihydroxid umsetzt und anschließend die Verbindung der Formel (cis-IV) bzw. (cis-II) abtrennt.

Als Verdünnungsmittel wird in dem erfindungsgemäßen Verfahren Wasser verwendet.

Als Alkali- oder Erdalkalihydroxid können bei der Durchführung des erfindungsgemäßen Verfahrens alle bekannten Alkali- oder Erdalkalihydroxide eingesetzt werden. Vorzugsweise verwendbar sind Lithium-, Natrium-, Kalium-, Caesium-, Magnesium-, Rubidium-, Calcium- oder Bariumhydroxid. Besonders bevorzugt sind Natrium-, Lithium und Kaliumhydroxid. Hervorgehoben sind Natrium- und Kaliumhydroxid.

Bei der Durchführung des erfindungsgemäßen Verfahrens setzt man das Hydantoin der Formel (II) und das Alkali- oder Erdalkalihydroxid im Allgemeinen in solchen molaren Verhältnissen ein, dass die molare Menge an Alkalihydroxid dem molaren Anteil an (cis-II) im Isomerengemisch (II) entspricht, bzw. dass die molare Menge an Erdalkalihydroxid halb so groß ist wie der molare Anteil an (cis-II) im Isomerengemisch (II) Es ist jedoch auch möglich, die eine oder andere Komponente in einem größeren Überschuss (bis zu 2 Mol) zu verwenden

Die Reaktionstemperatur kann bei der Durchführung des erfindungsgemäßen Verfahrens innerhalb eines größeren Bereiches variiert werden. Im Allgemeinen arbeitet man bei Temperaturen zwischen 0°C und 100°C, vorzugsweise zwischen 10°C und 90°C.

Das erfindungsgemäße Verfahren wird im Allgemeinen unter Normaldruck durchgeführt

Die Durchführung des erfindungsgemäßen Verfahrens und die Herstellung der erfindungsgemäßen Verbindungen geht aus den nachfolgenden Beispielen hervor, ohne durch sie eingeschränkt zu sein.

### Herstellungsbeispiele

### Beispiel 1 (mit LiOH)

Eine Suspension bestehend aus 19,84 g (0,1 Mol) der Verbindung (II, A = Me: Reinheit 99,9%ig; Isomerenverhältnis cis/trans = 75 : 25) und 1,857 g (0,076 Mol) LiOH (98%ig) in 48 ml Wasser wird 30 Minuten bei 80°C gerührt. Danach lässt man auf Raumtemperatur abkühlen, saugt den Feststoff ab, wäscht ihn mit 10 ml Wasser und trocknet. Man erhält 9,23 g der Verbindung (II) mit einem Isomerenverhältnis cis/trans von 99,9: 0,1.

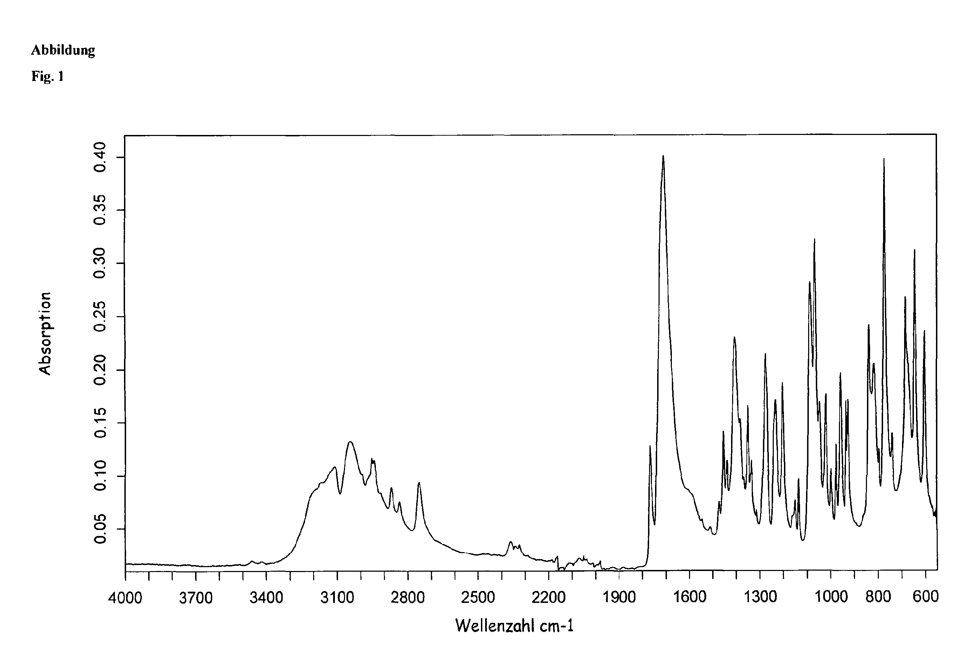

Das IR-Spektrum der Verbindung (cis-II) findet sich in Abbildung 1.

Intensive Banden: 1767 cm⁻¹, 1709 cm⁻¹, 1407 cm⁻¹, 1276 cm⁻¹, 1233 cm⁻¹, 1203 cm⁻¹, 1087 cm⁻¹, 1067 cm⁻¹, 8 3 6 c m⁻¹, 815 cm⁻¹, 771 cm⁻¹, 682 cm⁻¹, 642 cm⁻¹, 602 cm⁻¹.

### Beispiel 2 (mit NaOH)

Eine Suspension bestehend aus 19,84 g (0,1 Mol) der Verbindung (II, A = Me; Reinheit 99,9%ig; Isomerenverhältnis cis/trans = 75 : 25) und 3,04 g (0,076 Mol) NaOH in 48 ml Wasser wird 30 Minuten bei 80°C gerührt. Danach lässt man auf Raumtemperatur abkühlen, rührt für weitere 30 Minuten, saugt den Feststoff ab, wäscht ihn mit 10 ml Wasser und trocknet. Man erhält 12,26 g Feststoff mit einem Isomerenverhältnis cis/trans von 95,0 : 5,0 und einem Wassergehalt von 0,6 Gewichtsprozent.

Durch Ionenchromatographie wurde ein Natriumgehalt von 7,07 Gewichtsprozent festgestellt (theoret.: 10,4%); d.h. es liegt überwiegend ein Gemisch aus (cis-II) und (cis-IV) vor.

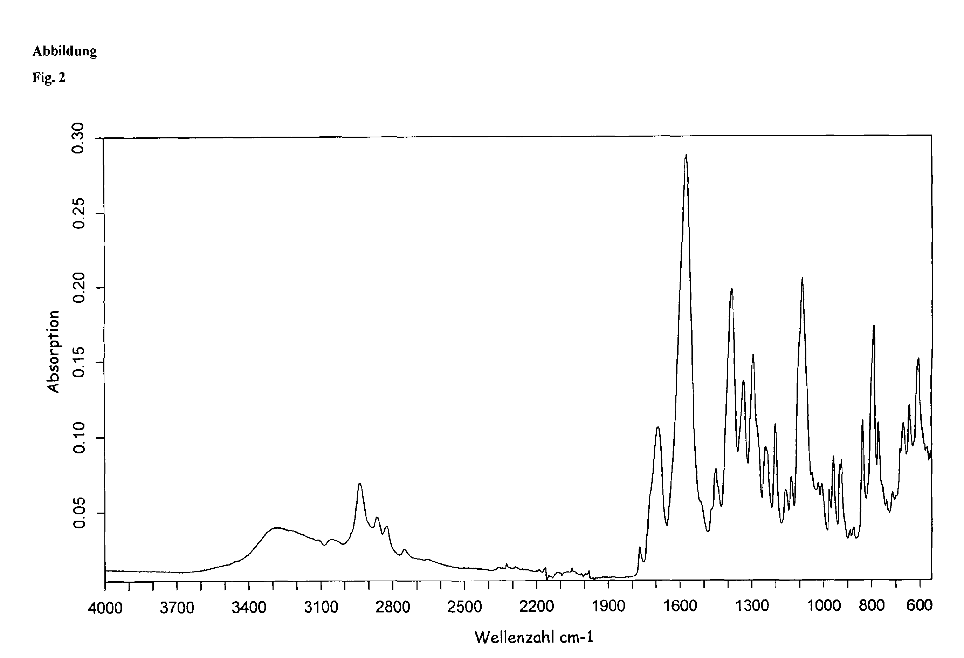

Das IR-Spektrum des Produktes zeigt die Abbildung 2.

Für das Natriumsalz (cis-IV, M= Na, n=1) sind folgende Banden charakteristisch:
1687 cm⁻¹, 1573 cm⁻¹, 1386 cm⁻¹, 1334 cm⁻¹, 1295 cm⁻¹, 1090 cm⁻¹, 793 cm⁻¹.

### Beispiel 3 (mit NaOH)

100 g eines cis/trans Rohgemisches der Verbindung II (mit A = Me), welches als Hauptkomponenten 66,9 Gew. % cis-Isomer und 22,6 Gew. % trans-Isomer enthält, wird zusammen mit 342 g einer 4%igen wässrigen Natronlauge auf 80 °C erwärmt. Ab ca. 70 °C bildet sich eine klare Lösung. Beim anschließenden Abkühlen der Lösung setzt bei ca. 45 °C spontane Kristallisation ein. Die Kristallsuspension wird noch weiter bis auf 20 °C abgekühlt. Der kristalline Feststoff wird abgesaugt, zweimal mit je 50 ml Wasser gewaschen und getrocknet.

Man erhält 43,5 g Feststoff mit einem Gehalt der Verbindungen II und IV (M = Na), der in Summe 91,0 Gew.% II entspricht, bei einem Isomerenverhältnis cis/trans von 97,5 : 2,5.

### Beispiel 4 (mit NaOH)

100 g eines cis/trans Rohgemisches der Verbindung II (mit A = Me), welches als Hauptkomponenten 66,9 Gew. % cis-Isomer und 22,6 Gew. % trans-Isomer enthält, wird zusammen mit 452 g einer 6 %eigen wässrigen Natronlauge auf 80 °C erwärmt. Ab ca. 60 °C bildet sich eine klare Lösung. Beim anschließenden Abkühlen der Lösung setzt bei ca. 50 °C spontane Kristallisation ein. Die Kristallsuspension wird noch weiter bis auf 20 °C abgekühlt. Der kristalline Feststoff wird abgesaugt, zweimal mit je 75 ml Wasser gewaschen und getrocknet. Man erhält 80,4 g Feststoff mit einem Gehalt der Verbindungen II und IV (M = Na), der in Summe 87,3 Gew.% II entspricht, bei einem Isomerenverhältnis cis/trans von 97,5 : 2,5.

### Beispiel 5 (mit NaOH)

100 g eines cis/trans Rohgemisches der Verbindung II (mit A = Me), welches als Hauptkomponenten 66,9 Gew. % cis-Isomer und 22,6 Gew. % trans-Isomer enthält, wird zusammen mit 484 g einer 7 %igen wässrigen Natronlauge auf 80 °C erwärmt. Bei ca. 70 °C bildet sich eine klare Lösung. Beim anschließenden Abkühlen der Lösung setzt bei ca. 50 °C spontane Kristallisation ein. Die Kristallsuspension wird noch weiter bis auf 20 °C abgekühlt. Der kristalline Feststoff wird abgesaugt, mit 150 ml Wasser gewaschen und getrocknet. Man erhält 76,4 g Feststoff mit einem Gehalt der Verbindungen II und IV (M = Na), der in Summe 90,6 Gew. % II entspricht, bei einem Isomerenverhältnis cis/trans von 96,9 : 3,1.

### Beispiel 6 (mit KOH)

Eine Suspension bestehend aus 19,84 g (0,1 Mol) der Verbindung (II, A = Me; Reinheit 99,9%ig; Isomerenverhältnis cis/trans = 75 : 25) und 5,016 g (0,076 Mol) KOH (85%ig) in 48 ml Wasser wird 30 Minuten bei 80°C gerührt. Danach lässt man auf Raumtemperatur abkühlen, rührt für weitere 30 Minuten, saugt den Feststoff ab, wäscht ihn mit 10 ml Wasser und trocknet. Man erhält 7,86 g Feststoff mit einem Isomerenverhältnis cis/trans von 98,3 : 1,7 und einem Wassergehalt von 10,28 Gewichtsprozent.

Durch Ionenchromatographie wurde ein Kaliumgehalt von 8,7 Gewichtsprozent festgestellt (theoret.: 16,55%): d.h. es liegt überwiegend ein Gemisch aus (cis-II) und (cis-IV) vor.

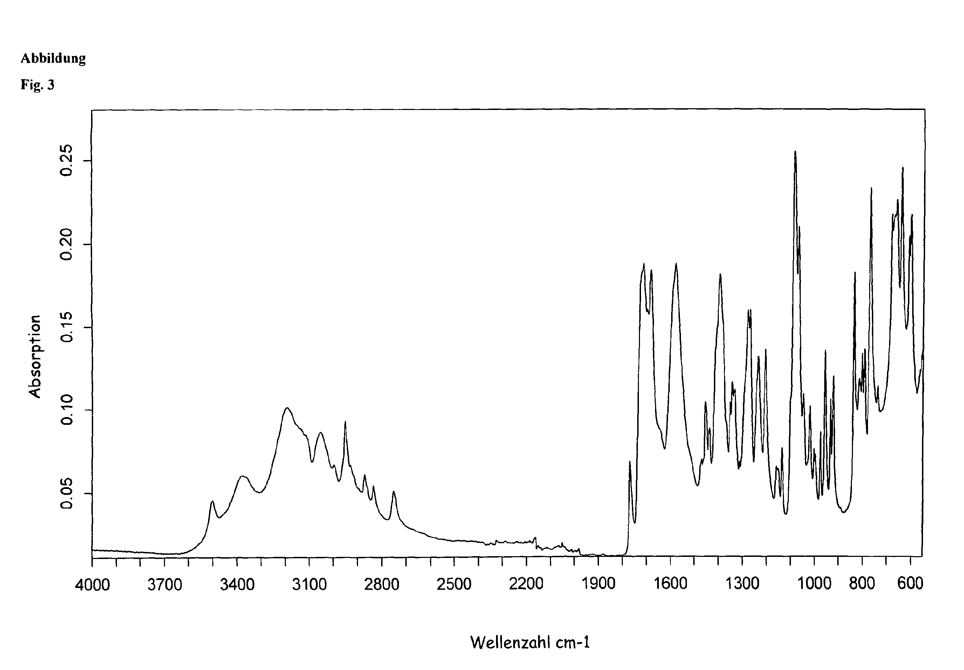

Das IR-Spektrum des Produktes zeigt die Abbildung 3.

Für das Kaliumsalz (cis-IV, M=K, n=1) sind folgende Banden charakteristisch:
1679 cm⁻¹, 1576 cm⁻¹, 1393 cm⁻¹. 1267 cm⁻¹.

### Beispiel 7 (mit RbOH)

Eine Suspension bestehend aus 19,84 g (0,1 Mol) der Verbindung (II, A = Me; Reinheit 99,9%ig; Isomerenverhältnis cis/trans = 75 : 25) und 15,73 g (0,076 Mol) RbOH (50%ig wässrige Lösung) in 40 ml Wasser wird 30 Minuten bei 80°C gerührt. Danach lässt man auf Raumtemperatur abkühlen, rührt für weitere 30 Minuten, saugt den Feststoff ab, wäscht ihn mit 10 ml Wasser und trocknet. Man erhält 3,63 g Feststoff mit einem Isomerenverhältnis cis/trans von 99,3 : 0,7 und einem Wassergehalt von 0,44 Gewichtsprozent.

Durch Elementaranaylse wurde ein Rubidiumgehalt von 0,17 Gewichtsprozent festgestellt (theoret.: 30,23%); d.h. es liegt praktisch ausschließlich (cis-II) vor.

### Beispiel 8 (mit CsOH)

Eine Suspension bestehend aus 19,84 g (0,1 Mol) der Verbindung (II, A = Me; Reinheit 99,9%ig; Isomerenverhältnis cis/trans = 75 : 25) und 23,02 g (0,076 Mol) CsOH (50%ig wässrige Lösung) in 36 ml Wasser wird 30 Minuten bei 80°C gerührt. Danach lässt man auf Raumtemperatur abkühlen, rührt für weitere 30 Minuten, saugt den Feststoff ab, wäscht ihn mit 10 ml Wasser und trocknet. Man erhält 3,78 g Feststoff mit einem Isomerenverhältnis cis/trans von 99,2 : 0,8 und einem Wassergehalt von 0,11 Gewichtsprozent.

Durch Elementaranaylse wurde ein Cesiumgehalt von 0,18 Gewichtsprozent festgestellt (theoret.: 40,26%); d.h. es liegt praktisch ausschließlich (cis-II) vor.

### Beispiel 9 (mit Ca(OH)₂)

Eine Suspension bestehend aus 19,84 g (0,1 Mol) der Verbindung (II, A = Me; Reinheit 99,9%ig; Isomerenverhältnis cis/trans = 75 : 25) und 2,873 g (0,038 Mol) Ca(OH)₂ (98%ig) in 48 ml Wasser wird 30 Minuten bei 80°C gerührt. Danach gibt man weitere 15 ml Wasser hinzu, lässt auf Raumtemperatur abkühlen, rührt für weitere 30 Minuten, saugt den Feststoff ab, wäscht ihn mit 10 ml Wasser und trocknet. Man erhält 16,98 g Feststoff mit einem Isomerenverhältnis cis/trans von 88 : 12 und einem Wassergehalt von 3,08 Gewichtsprozent.

Durch Elementaranaylse wurde ein Calciumgehalt von 5,7 Gewichtsprozent festgestellt (theoret.: 9,2%); d.h. es liegt überwiegend ein Gemisch aus (cis-II) und (cis-IV) vor.

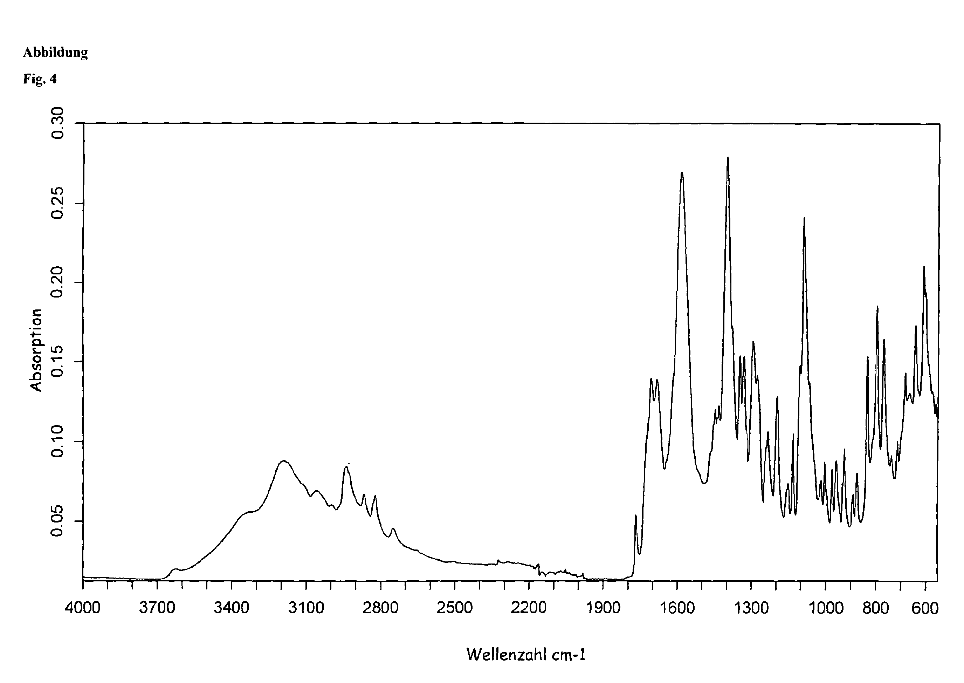

Das IR-Spektrum des Produktes zeigt die Abbildung 4.

Für das Calciumsalz (cis-IV, M=Ca, n=2) sind folgende Banden charakteristisch:
1682 cm⁻¹, 1583 cm⁻¹, 1399 cm⁻¹, 1294 cm⁻¹, 797 cm⁻¹.

### Beispiel 10 (mit Ba(OH)₂)

Eine Suspension bestehend aus 19,84 g (0,1 Mol) der Verbindung (II, A = Me; Reinheit 99,9%ig; Isomerenverhältnis cis/trans = 75 : 25) und 12,358 g (0,038 Mol) Ba(OH)₂-Octahydrat (97%ig) in 42 ml Wasser wird 30 Minuten bei 80°C gerührt. Danach gibt man weitere 15 ml Wasser hinzu, lässt auf Raumtemperatur abkühlen, rührt für weitere 30 Minuten, saugt den Feststoff ab, wäscht ihn mit 10 ml Wasser und trocknet. Man erhält 16,98 g Feststoff mit einem Isomerenverhältnis cis/trans von 94,2 : 5,8 und einem Wassergehalt von 10,86 Gewichtsprozent.

Durch Elementaranaylse wurde ein Bariumgehalt von 14 Gewichtsprozent festgestellt (theoret.: 25,83%); d.h. es liegt überwiegend ein Gemisch aus (cis-II) und (cis-IV) vor.

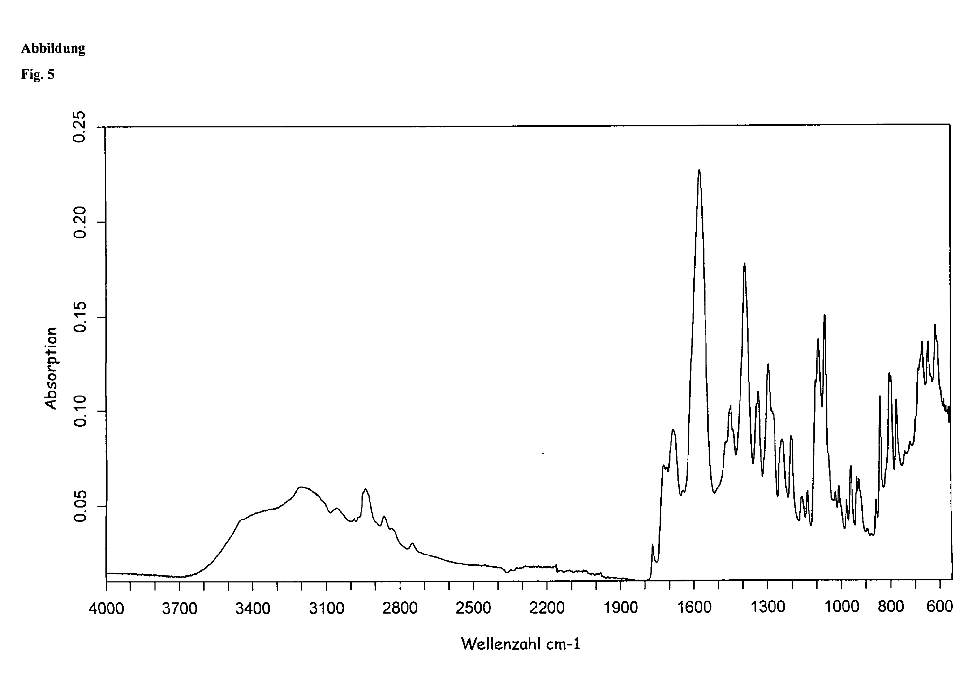

Das IR-Spektrum des Produktes zeigt die Abbildung 5.

Für das Bariumsalz (cis-IV, M=Ba, n=2) sind folgende Banden charakteristisch:
1682 cm⁻¹, 1573 cm⁻¹, 1389 cm⁻¹, 1293 cm⁻¹, 799 cm⁻¹.

## Patentansprüche

1. Verfahren zur Isolierung von cis-Hydantoinen der Formel (cis-II) 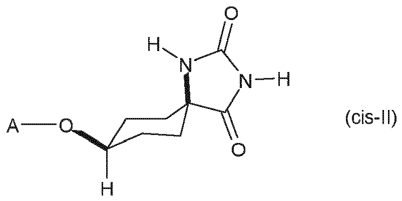 in welcher
A für C₁-C₆-Alkyl steht,
**dadurch gekennzeichnet, dass** man ein Isomerengemisch der Formel (II) 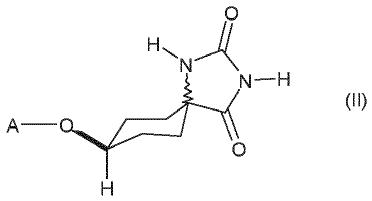 in welcher A die oben genannte Bedeutung hat
mit einer wässrigen Lösung eines Alkali- oder Erdalkalihydroxids verrührt und anschließend das cis-Isomer isoliert.

2. Verfahren gemäß Anspruch 1, wobei A für C₁ - C₄-Alkyl steht.

3. Verfahren gemäß Anspruch 1, wobei A für Methyl, Ethyl, Propyl, i-Propyl, Butyl oder i-Butyl steht.

4. Verahren gemäß Anspruch 1, wobei A für Methyl steht.

5. Verfahren gemäß Anspruch 1, **dadurch gekennzeichnet, dass** Lithium-, Natrium-, Kalium-, Caesium-, Magnesium-, Rubidium-, Calcium- oder Bariumhydroxid eingesetzt werden.

6. Verbindungen der Formel (cis-IV) 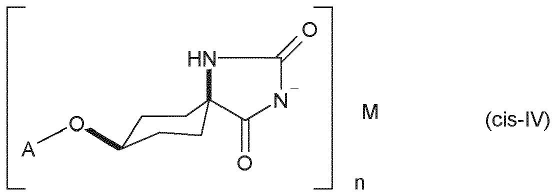 in welcher
A für C₁ - C₆-Alkyl steht,
M für ein Alkalikation oder für ein Erdalkalikation steht, und
n für 1 steht, wenn M ein Alkalikation ist
oder
für 2 steht, wenn M ein Erdalkalikation ist.

7. Verbindungen der Formel (cis-IV) gemäß Anspruch 6,
in welcher
A für Methyl steht,
M für Li⁺, Na⁺ oder K⁺ steht und
n für 1 steht.

8. Verbindungen der Formel (cis-IV) gemäß Anspruch 6, in welcher
M für Na⁺ steht und
n für 1 steht.

9. Hydrate der Verbindungen der Formel (cis-IV) gemäß Anspruch 6.

## Claims

1. Process for isolating cis-hydantoins of the formula (cis-II) 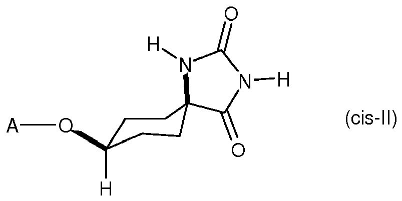 in which
A represents C₁-C₆-alkyl,
**characterized in that** an isomer mixture of the formula (II) 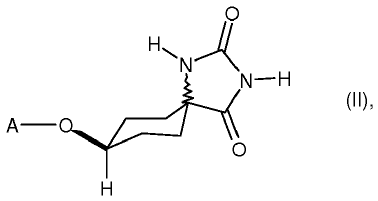 in which A has the meaning mentioned above,
is stirred with an aqueous solution of an alkali metal hydroxide or alkaline earth metal hydroxide and the cis isomer is then isolated.

2. Process according to Claim 1, where A represents C₁-C₄-alkyl.

3. Process according to Claim 1, where A represents methyl, ethyl, propyl, isopropyl, butyl or isobutyl.

4. Process according to Claim 1, where A represents methyl.

5. Process according to Claim 1, **characterized in that** lithium hydroxide, sodium hydroxide, potassium hydroxide, cesium hydroxide, magnesium hydroxide, rubidium hydroxide, calcium hydroxide or barium hydroxide is used.

6. Compounds of the formula (cis-IV) 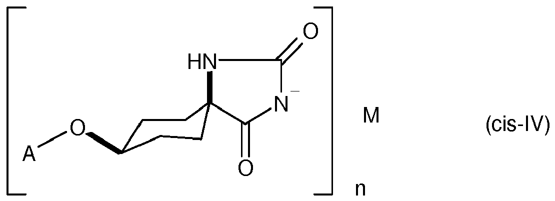 in which
A represents C₁-C₆-alkyl,
M represents an alkali metal cation or represents an alkaline earth metal cation and
n represents 1 if M is an alkali metal cation
or
represents 2 if M is an alkaline earth metal cation.

7. Compounds of the formula (cis-IV) according to Claim 6
in which
A represents methyl,
M represents Li⁺, Na⁺ or K⁺ and
n represents 1.

8. Compounds of the formula (cis-IV) according to Claim 6 in which
M represents Na⁺ and
n represents 1.

9. Hydrates of the compounds of the formula (cis-IV) according to Claim 6.

## Revendications

1. Procédé pour l'isolement de cis-hydantoïnes de formule (cis-II) 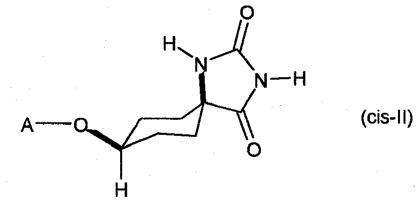 dans laquelle
A représente alkyle en C₁-C₆,
**caractérisé en ce qu'**on mélange en remuant un mélange d'isomères de formule (II) 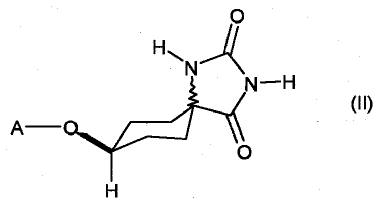 dans laquelle A présente la signification susmentionnée avec une solution aqueuse d'un hydroxyde de métal alcalin ou de métal alcalino-terreux et on isole ensuite l'isomère cis.

2. Procédé selon la revendication 1, A représentant alkyle en C₁-C₄.

3. Procédé selon la revendication 1, A représentant méthyle, éthyle, propyle, i-propyle, butyle ou i-butyle.

4. Procédé selon la revendication 1, A représentant méthyle.

5. Procédé selon la revendication 1, **caractérisé en ce qu'**on utilise de l'hydroxyde de lithium, de sodium, de potassium, de césium, de magnésium, de rubidium, de calcium ou de baryum.

6. Composés de formule (cis-IV) 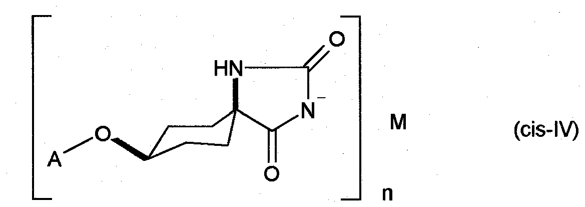 dans laquelle
A représente alkyle en C₁-C₆,
M représente un cation de métal alcalin ou un cation de métal alcalino-terreux et
n vaut 1 lorsque M représente un cation de métal alcalin ou vaut 2 lorsque M représente un cation de métal alcalino-terreux.

7. Composés de formule (cis-IV) selon la revendication 6, dans laquelle
A représente méthyle,
M représente Li⁺, Na⁺ ou K⁺ et
n vaut 1.

8. Composés de formule (cis-IV) selon la revendication 6, dans laquelle
M représente Na⁺ et
n vaut 1.

9. Hydrates des composés de formule (cis-IV) selon la revendication 6.
